# EUROPEAN PATENT APPLICATION

(11) **EP 4 074 290 A1**
(43) Date of publication of application: **19.10.2022**
(21) Application number: 22168223.0
(22) Date of filing: 13.04.2022
(51) Int. Cl.: A61F 5/02, B25J 9/00

(54) **ASSISTIVE EQUIPMENT AND SUPPORTING DEVICE OF THE SAME**

(30) Priority: 15.04.2021 US 202163175206 P
(71) Applicant: Free Bionics Taiwan Inc., 300 Hsinchu City (TW)
(72) Inventor: Tsai, Yi-Jeng, 330 Taoyuan City (TW); Teng, Ming-Chang, 300 Hsinchu City (TW); Fan, Yu-Hua, 300 Hsinchu City (TW)
(74) Representative: Michalski Hüttermann & Partner Patentanwälte mbB

(57) **Abstract**

The present disclosure provides an assistive equipment and a supporting device of the same. The supporting device includes a housing, a first positioning element, a second positioning element and an elastic element. The first positioning element and the second positioning element are respectively disposed at two ends of the housing. The elastic element is disposed between the first positioning element and the second positioning element. When the second positioning element positions an end of the elastic element, a rotation of the second positioning element at a first pivot produce a deformation to the elastic element.

## Description

### BACKGROUND OF THE PRESENT DISCLOSURE

### FIELD OF THE PRESENT DISCLOSURE

The present disclosure relates to an assistive equipment and a supporting device of the same and, more particularly, to an assistive equipment and a supporting device of the same, which provide a supporting force to a user's body.

### DESCRIPTION OF THE PRIOR ART

Thanks to technological advancement, various tools that assist with movement of the human body are available to enable users to perform related motion easily. However, conventional tool designs are not only complicated but also require overabundant operation steps, to the detriment of intuitive use. In view of this, it is necessary to provide good assistive tools conducive to enhancement of efficiency and ease of use.

### SUMMARY OF THE PRESENT DISCLOSURE

In view of the aforesaid drawbacks of the prior art, it is an objective of the present disclosure to provide an assistive equipment and a supporting device of the same, which provide a supporting force to a user's body.

In order to achieve the above and other objectives, the present disclosure provides a supporting device comprising a housing, a first positioning element, a second positioning element and an elastic element. The first positioning element and the second positioning element are disposed at two ends of the housing, respectively. The elastic element is disposed between the first positioning element and the second positioning element. When an end of the elastic element is positioned in place by the second positioning element, rotation of the first positioning element about a first pivoting point causes a deformation of the elastic element.

The present disclosure further provides an assistive equipment comprising the supporting device, a wearing unit, a supporting unit and a positioning unit. The wearing unit is connected to the at least one supporting device to allow the at least one supporting device to be worn on a user's body. The supporting unit is connected to the first positioning element of the at least one supporting device and adapted to support a top half of the body of the user. The positioning unit is connected to the housing of the at least one supporting device and adapted to position the at least one supporting device in place.

### BRIEF DESCRIPTION OF THE DRAWINGS

The embodiments of the present disclosure are illustrated by accompanying drawings and described below. The accompanying drawings are not drawn to scale; thus, for the sake of illustration, features shown in the accompanying drawings may be enlarged or diminished as needed.
FIG. 1A is a perspective view of a supporting device according to some embodiments of the present disclosure.
FIG. 1B is a side view of the supporting device according to some embodiments of the present disclosure.
FIG. 1C is a schematic view of internal structure of the supporting device according to some embodiments of the present disclosure.
FIG. 1D is a schematic view of internal structure of the supporting device according to some embodiments of the present disclosure.
FIG. 1E is a schematic view of the supporting device operating according to some embodiments of the present disclosure.
FIG. 2A is a perspective view of a supporting device according to some embodiments of the present disclosure.
FIG. 2B is a side view of the supporting device according to some embodiments of the present disclosure.
FIG. 2C is a schematic view of internal structure of the supporting device according to some embodiments of the present disclosure.
FIG. 2D is a perspective view of the supporting device according to some embodiments of the present disclosure.
FIG. 2E is a side view of the supporting device according to some embodiments of the present disclosure.
FIG. 2F is a schematic view of internal structure of the supporting device according to some embodiments of the present disclosure.
FIG. 2G is a schematic view of the supporting device operating according to some embodiments of the present disclosure.
FIG. 2H is a schematic view of the supporting device operating according to some embodiments of the present disclosure.
FIG. 3A is a perspective view of an assistive equipment according to some embodiments of the present disclosure.
FIG. 3B is a schematic view of a user using the assistive equipment according to some embodiments of the present disclosure.
FIG. 3C is a schematic perspective view of a supporting unit connected to a connecting unit according to some embodiments of the present disclosure.
FIG. 3D is a schematic side view of the supporting unit connected to the connecting unit according to some embodiments of the present disclosure.
FIG. 3E is a schematic perspective view of a positioning unit connected to the connecting unit according to some embodiments of the present disclosure.

### DETAILED DESCRIPTION OF THE EMBODIMENTS

Different embodiments and examples of different features of the subject matters of the present disclosure are disclosed below. Specific embodiments of constituent elements of the present disclosure and arrangement thereof serve an illustrative rather than restrictive purpose. According to the present disclosure, an embodiment in which a first feature formed above or on a second feature may be interpreted to mean that the first feature and the second feature are in direct contact with each other or that an additional feature is formed between the first feature and the second feature to preclude direct contact between the first feature and the second feature. Furthermore, reference numerals and/or alphabets are repeated in various embodiments to attain conciseness and clarification rather than indicate any relationship between the embodiments and/or examples.

The embodiments of the present disclosure are described in detail below. However, the present disclosure provides applicable concepts which can be embodied widely in various specific situations. The specific embodiments are illustrative rather than restrictive of the scope of the present disclosure.

For the sake of illustration, space-related expressions, such as "below", "under", "above", "top", "bottom", "left" and "right", are descriptive of the relationship between an element (or feature) and the other (or a plurality of) elements (or features) shown in the diagrams. In addition to the directions indicated in the diagrams, the space-related expressions are intended to specify different directions related to the device in use or in operation. The equipment may operate in any other directions (rotate by 90 degrees or lie in any other direction.) Likewise, the space-related expressions may be interpreted accordingly. When an element disclosed herein is referred to as "connected to" or "coupled to" another element, the element may be connected or coupled to another element either directly or indirectly through an intervening element.

All numeric value ranges and parameters used herein are approximations which indicate numeric values in the specific embodiments of the present disclosure as precisely as possible. However, some of the numeric values may contain errors inevitably arising from standard deviations found in each measurement. Furthermore, the term "around" usually refers to a given value or range with a ±10%, ±5%, ±1% or ±0.5% error. Alternatively, average persons skilled in the art believe that the term "around" refers to a given value which is within an acceptable standard deviation of the mean. Except in an example of operation/duty or unless otherwise expressly specified, numeric value ranges, levels, values and percentages (such as material quantity, duration, temperature, operation criteria, and ratios) can be described with the term "around" in all situations. Therefore, unless otherwise specified, the numeric values and parameters disclosed in the present disclosure and the appended claims are approximations which are subject to changes. At the very least, the numeric values and parameters must be interpreted in accordance with the disclosed significant figures and the general rule for rounding. The ranges disclosed herein each start at one end point and end at the other end point or are each defined between two end points. Unless otherwise specified, every range disclosed herein includes end points. The term "substantially coplanar" refers to two surfaces lying on the same plane and separated by a distance of several micrometres (µm) or less (for example, lying on the same plane and separated by a distance of 10 µm or less, 5 µm or less, 1 µm or less, or 0.5 µm or less). When numeric values or features are considered "substantially" equal, the term "substantially" means that the numeric values are within a ±10%, ±5%, ±1% or ±0.5% error range of the mean of the numeric values.

The present disclosure provides a supporting device and an assistive equipment using the supporting device. The assistive equipment comprises a wearing unit for wearing the supporting device on a user's body. The user operates the supporting device according to the user's motion, such as stooping, and the supporting device is conducive to the generation and transmission of a supporting force corresponding to the user's motion. When the user restores his or her initial posture from the motion (for example, switches from stooping to standing), the supporting device assists the user in restoring his or her initial posture under the supporting force.

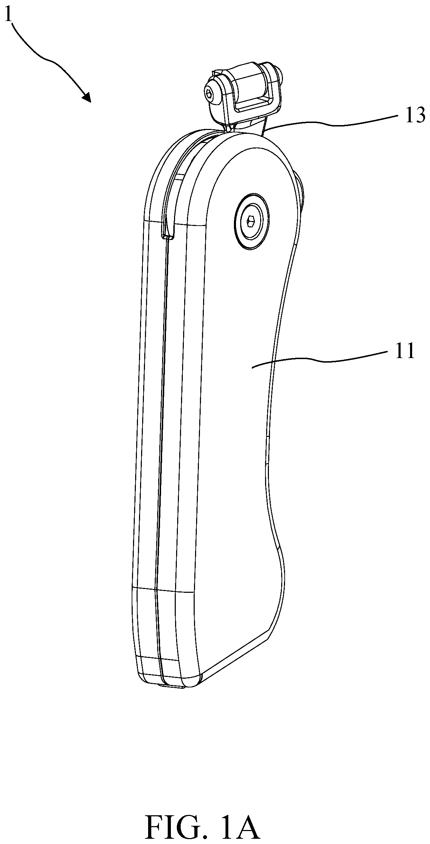

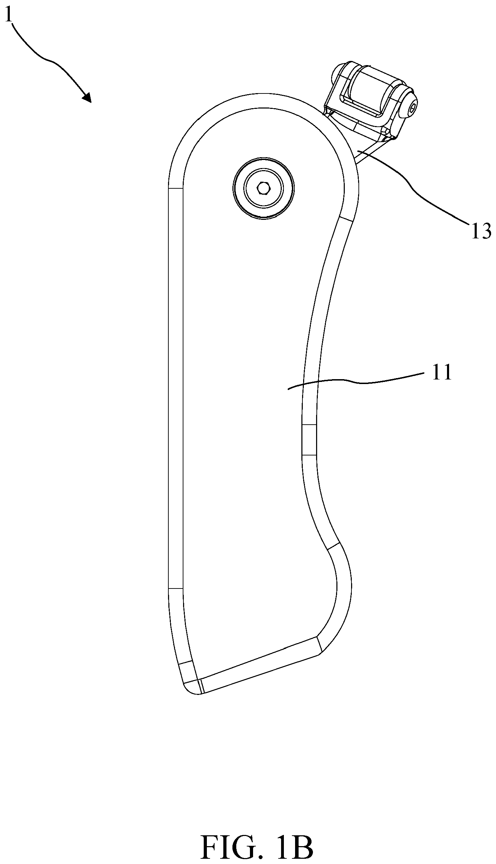

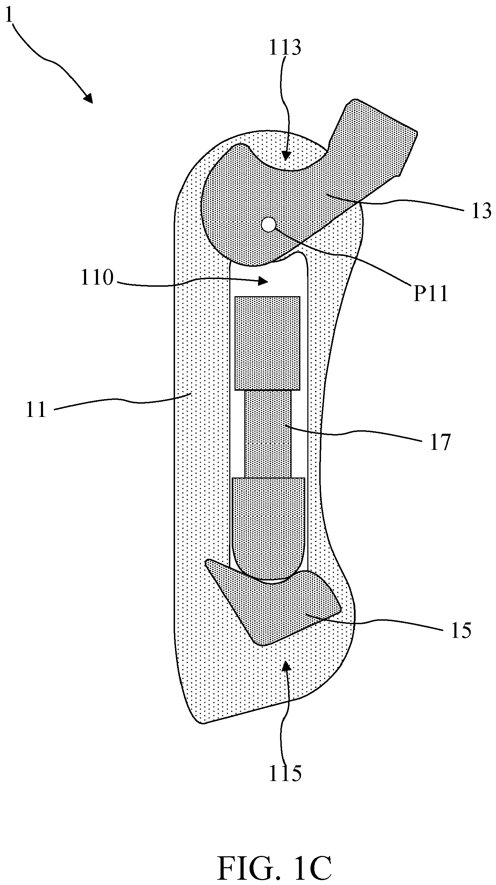

Refer to FIGs. 1A, 1B and 1C. FIG. 1A is a perspective view of a supporting device 1 according to some embodiments of the present disclosure. FIG. 1B is a side view of the supporting device 1 according to some embodiments of the present disclosure. FIG. 1C is a schematic view of internal structure of the supporting device 1 according to some embodiments of the present disclosure. The supporting device 1 comprises a housing 11, a first positioning element 13, a second positioning element 15 and an elastic element 17. The first positioning element 13 and the second positioning element 15 are disposed at two ends 113, 115 of the housing 11, respectively. The housing 11 has an internal space 110. The elastic element 17 is disposed in the internal space 110 and between the first positioning element 13 and the second positioning element 15.

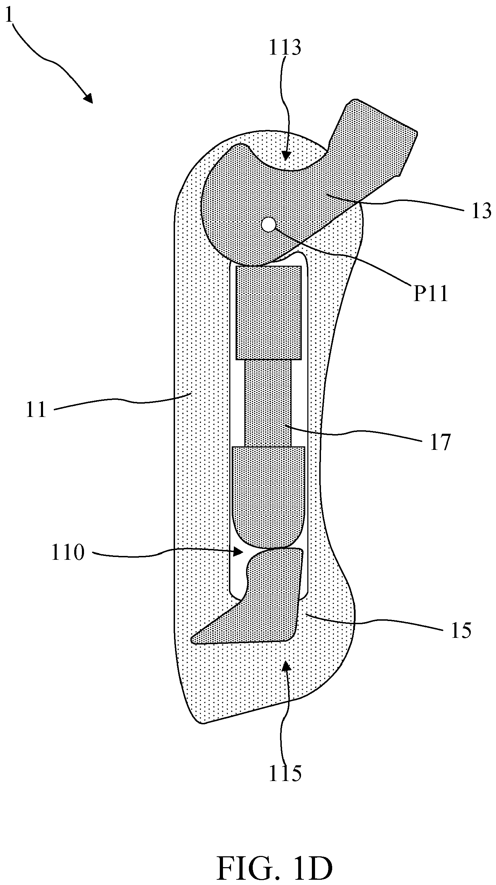

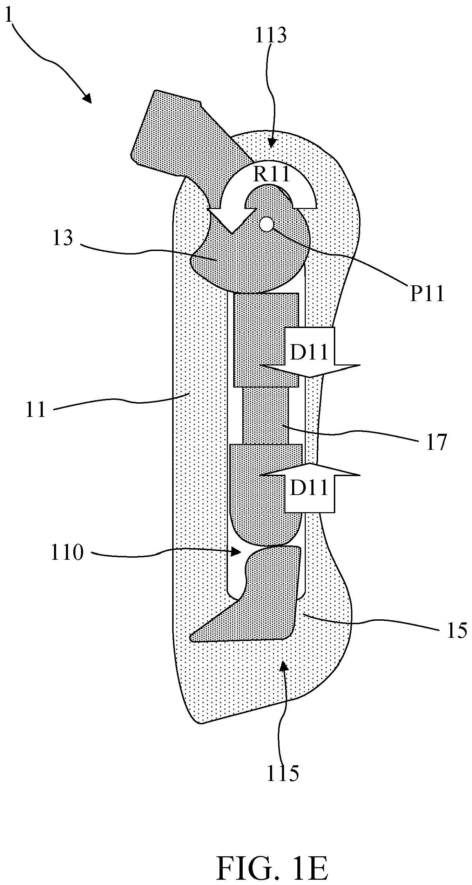

Refer to FIG. 1D and FIG. 1E. FIG. 1D is another schematic view of internal structure of the supporting device 1 according to some embodiments of the present disclosure. FIG. 1E is a schematic view of the supporting device 1 operating according to some embodiments of the present disclosure. As shown in FIG. 1D, one end of the elastic element 17 is positioned in place by the second positioning element 15 to reduce the slidable range of the elastic element 17 between the first positioning element 13 and the second positioning element 15. Then, as shown in FIG. 1E, rotation R11 of the first positioning element 13 about first pivoting point P11 causes deformation D11 of the elastic element 17. In some embodiments, deformation D11 of the elastic element 17 is compression deformation.

Given the aforesaid mechanism, during its rotation R11, the first positioning element 13 compresses the elastic element 17, and thus a restoring force generated by the elastic element 17 being compressed opposes the rotation R11 of the first positioning element 13. Therefore, when the rotation R11 occurs to the first positioning element 13 supporting the user bending his or her body, the restoring force generated by the elastic element 17 also serves to support the user's body.

For example, the first positioning element 13 undergoes the rotation R11 as soon as the first positioning element 13 supports the user's body while the user is stooping, and thus the restoring force opposing the rotation R11 of the first positioning element 13 and supporting the top half of the body of the user also serves to help the user restore the body's posture thereafter.

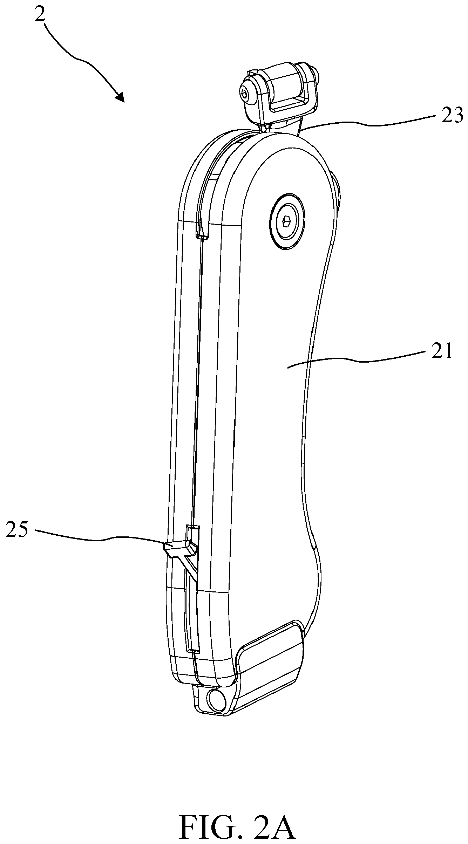

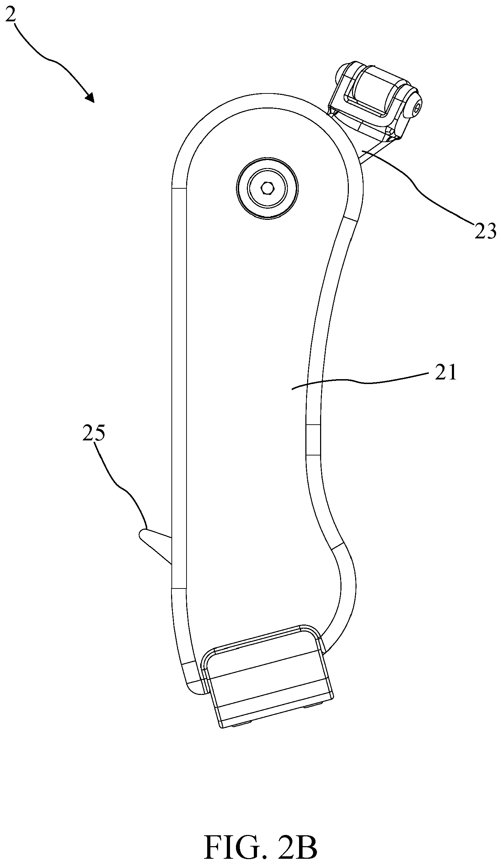

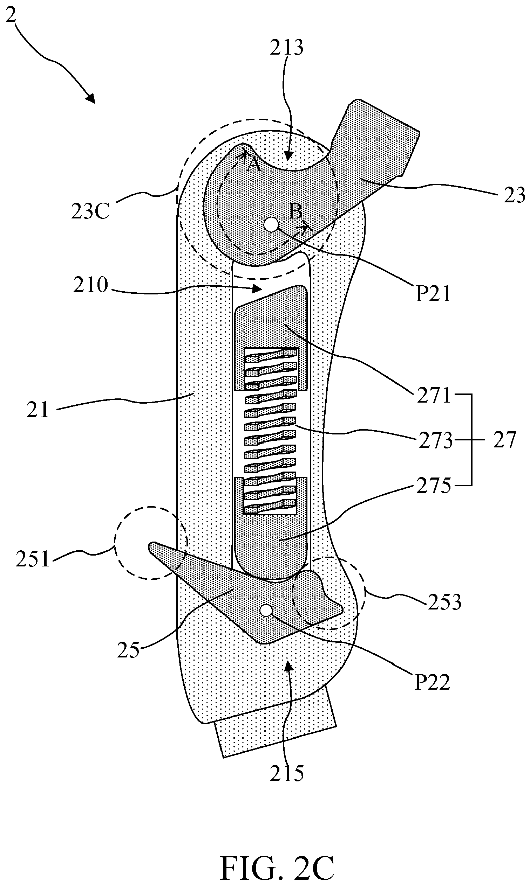

Refer to FIGs. 2A, 2B and 2C. FIG. 2A is a perspective view of a supporting device 2 according to some embodiments of the present disclosure. FIG. 2B is a side view of the supporting device 2 according to some embodiments of the present disclosure. FIG. 2C is a schematic view of internal structure of the supporting device 2 according to some embodiments of the present disclosure. The supporting device 2 comprises a housing 21, a first positioning element 23, a second positioning element 25 and an elastic element 27. The first positioning element 23 and the second positioning element 15 are disposed at two ends 213, 215 of the housing 21, respectively. The housing 21 has an internal space 210. The elastic element 27 is disposed in the internal space 210 and between the first positioning element 23 and the second positioning element 25.

In some embodiments, the first positioning element 23 comprises a cam 23C pivotally connected to a pivoting point P21 at an end 213 of the housing 21 and rotating about the pivoting point P21. The second positioning element 25 comprises an operating portion 251 and a positioning portion 253 pivotally connected to a pivoting point P22 at an end 215 of the housing 21 and rotating about the pivoting point P22. In some embodiments, the first positioning element 23 protrudes out of the housing 21 in the direction away from the cam 23C to connect to the supporting unit for supporting the user's body. The operating portion 251 of the second positioning element 25 protrudes out of the housing 21 so as to be operated by the user.

In some embodiments, the elastic element 27 comprises a first end portion 271, an elastomer 273 and a second end portion 275. The elastomer 273 connects the first end portion 271 and the second end portion 275. In the internal space 210, the first end portion 271 faces the first positioning element 23, and the second end portion 275 faces the second positioning element 25. The elastomer 273 is a coil spring, a stacked disc spring or a nitrogen spring; however, the present disclosure is not limited thereto. Thus, the elastomer 273 can be made of any elastic materials capable of compression and extension.

Referring to FIG. 2A through FIG. 2C, when the operating portion 251 operates the second positioning element 25 to allow the second positioning element 25 to rotate about the pivoting point P22 so as to be in a first state, the first positioning element 23 and the second positioning element 25 define a space range in the internal space 210 of the housing 21. In the space range, the elastic element 27 slides between the first positioning element 23 and the second positioning element 25, and the rotation of the first positioning element 23 about the pivoting point P21 does not cause any deformation of the elastic element 27. In some embodiments, when the operating portion 251 operates the second positioning element 25 to allow the second positioning element 25 to rotate about the pivoting point P22 so as to be in the first state, the second positioning element 25 stays in the first state.

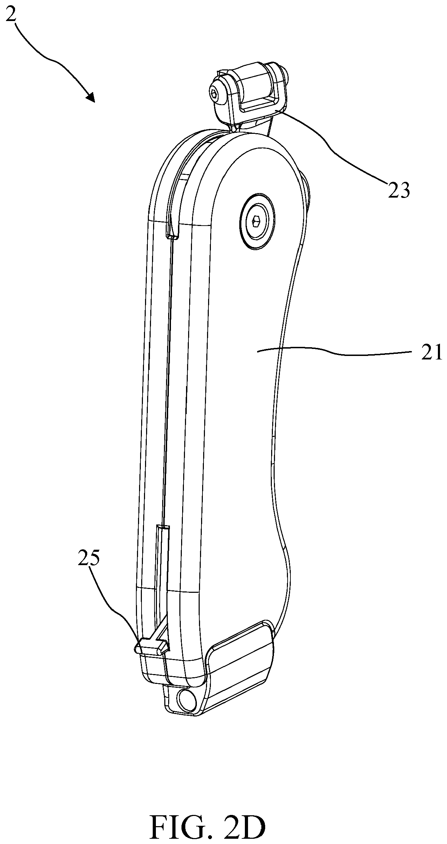

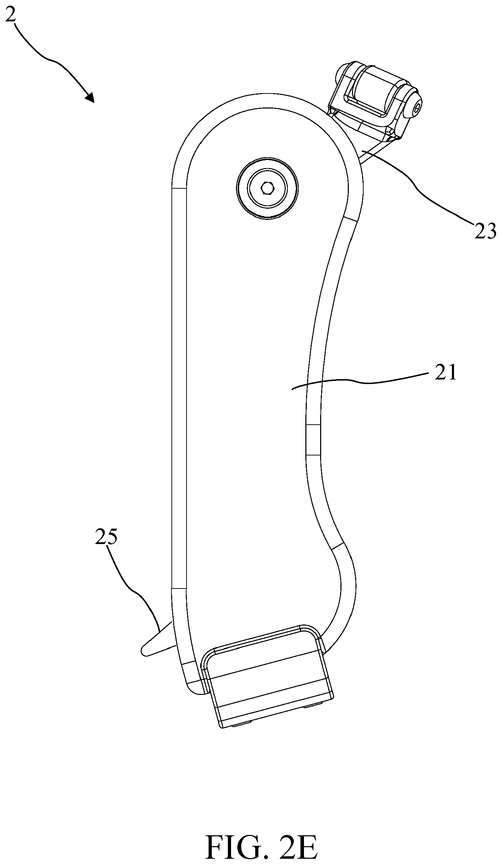

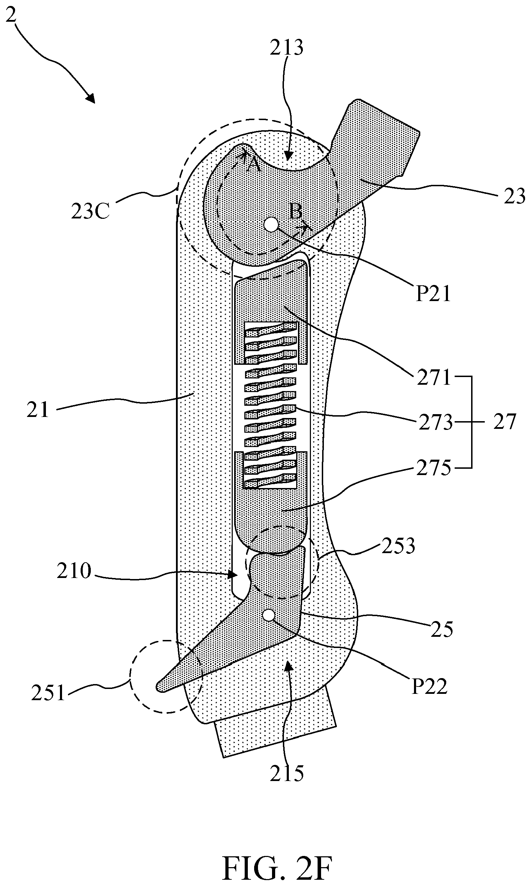

FIG. 2D is another perspective view of the supporting device 2 according to some embodiments of the present disclosure. FIG. 2E is another side view of the supporting device 2 according to some embodiments of the present disclosure. FIG. 2F is another schematic view of internal structure of the supporting device 2 according to some embodiments of the present disclosure. As shown in FIG. 2D, FIG. 2E, and FIG. 2F, when the operating portion 251 operates the second positioning element 25 to allow the second positioning element 25 to rotate about the pivoting point P22 so as to be in a second state, the second end 275 of the elastic element 27 is positioned in place by the positioning portion 253 of the second positioning element 25, thereby reducing the slidable range of the elastic element 27 between the first positioning element 23 and the second positioning element 25. In some embodiments, when the operating portion 251 operates the second positioning element 25 to allow the second positioning element 25 to rotate about the pivoting point P22 so as to be in the second state, the second positioning element 25 stays in the second state.

When the operating portion 251 operates the second positioning element 25 to allow the second positioning element 25 to rotate about the pivoting point P22 so as to be in the second state, not only does the positioning portion 253 of the second positioning element 25 abut against the second end 275 of the elastic element 27, but the elastic element 27 also gets closer to the second positioning element 25, thereby reducing the slidable range of the elastic element 27 between the first positioning element 23 and the second positioning element 25. In some embodiments, when the operating portion 251 operates the second positioning element 25 to allow the second positioning element 25 to rotate about the pivoting point P22 so as to be in the second state, the elastic element 27 abuts against the cam 23C at the base circle radius in an operable range of the cam 23C (from point A to point B, as indicated by dashed lines.) In some embodiments, when the operating portion 251 operates the second positioning element 25 to allow the second positioning element 25 to rotate about the pivoting point P22 so as to be in the second state, there is a distance between the elastic element 27 and the base circle radius in the operable range of the cam 23C.

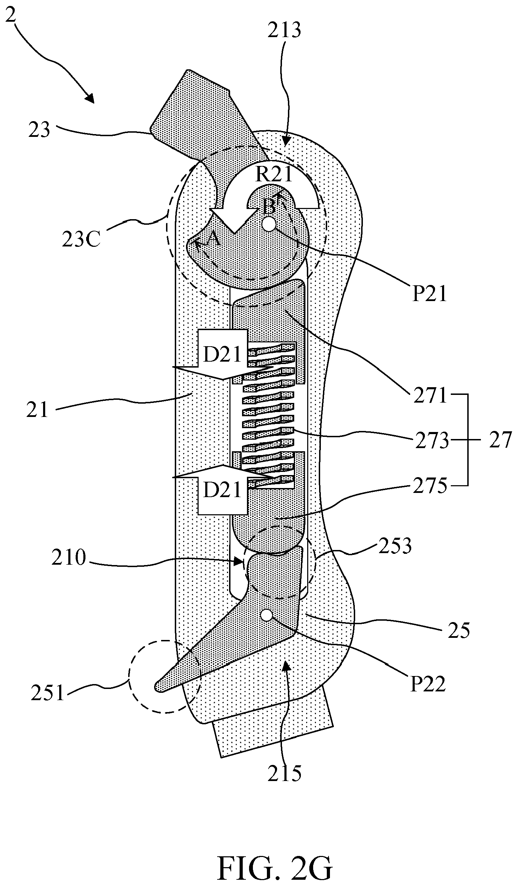

As shown in FIG. 2G, when the first positioning element 23 undergoes a rotation R21 about the first pivoting point P21, the cam 23C of the first positioning element 23 abuts against the first end portion 271 of the elastic element 27 in the course of the rotation R21 and presses against the first end portion 271 of the elastic element 27 in the early stage of the rotation R21, thereby causing deformation D21 of the elastomer 273 of the elastic element 27. In some embodiments, the deformation D21 of the elastomer 273 is compression deformation. In some embodiments, the radius in the operable range of the cam 23C increases progressively from the base circle radius to allow the torque for pressing against the first end portion 271 of the elastic element 27 in the course of the rotation R21 to increase progressively with the radius until the torque reaches the end (for example, point A in the operable range) of the operable range of the cam 23C.

In some embodiments, when the operating portion 251 operates the second positioning element 25 to allow the second positioning element 25 to rotate about the pivoting point P22 so as to be in the second state and allow the elastic element 27 to directly abut against the cam 23C at the base circle radius in the operable range of the cam 23C, the cam 23C directly presses against the first end portion 271 of the elastic element 27 during the early stage of the rotation R21 so as to cause the deformation D21 of the elastomer 273 of the elastic element 27.

In some embodiments, when the operating portion 251 operates the second positioning element 25 to allow the second positioning element 25 to rotate about the pivoting point P22 so as to be in the second state and allow for a distance between the elastic element 27 and the base circle radius in the operable range of the cam 23C, the cam 23C does not press against the elastic element 27 during the early stage of the rotation R21. It is only when the cam 23C abuts against the first end portion 271 of the elastic element 27 in the course of the rotation R21 that the first end portion 271 of the elastic element 27 can be compressed at the late stage of the rotation R21 to cause the deformation D21 of the elastomer 273 of the elastic element 27.

Given the aforesaid mechanism, during its rotation R21, the first positioning element 23 compresses the elastic element 27, and thus a restoring force generated by the elastic element 27 being compressed opposes the rotation R21 of the first positioning element 23. Therefore, when the rotation R21 occurs to the first positioning element 23 supporting the user bending his or her body, the restoring force generated by the elastic element 27 also serves to support the user's body.

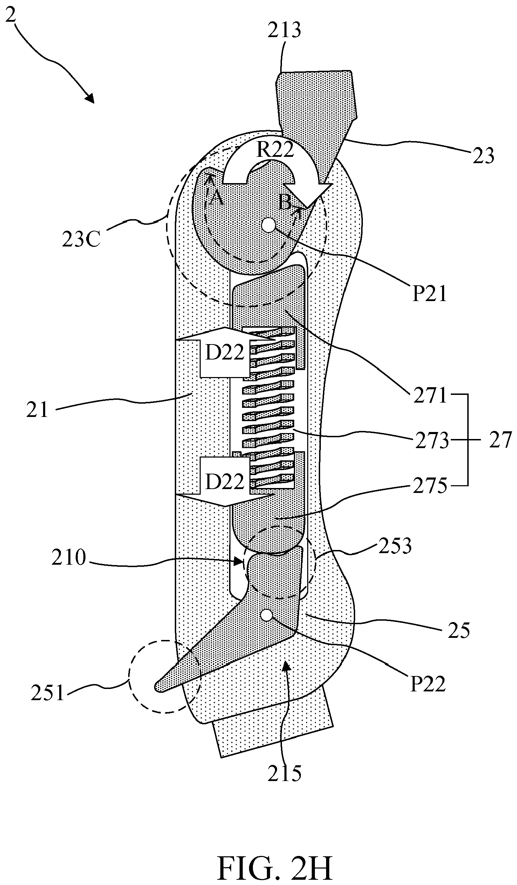

In some embodiments, when the user does not exert any force on the first positioning element 23, the first positioning element 23 undergoes a rotation in a direction opposite to that of the rotation R21 under a restoring force generated by the elastic element 27 restored from a compression state. Referring to FIG. 2H, there is shown another schematic view of the supporting device 2 operating according to some embodiments of the present disclosure. When the user does not exert any force on the first positioning element 23, the elastic element 27 is no longer subjected to the compression force from the cam 23C, allowing the elastic element 27 to restore from the compression state and undergo a deformation D22 (i.e., extension deformation). At this point in time, the elastic element 27 presses against the cam 23 so that the first positioning element 23 undergoes a rotation R22 in a direction opposite to that of the rotation R21. The rotation R22 assists the user in restoring his or her initial posture.

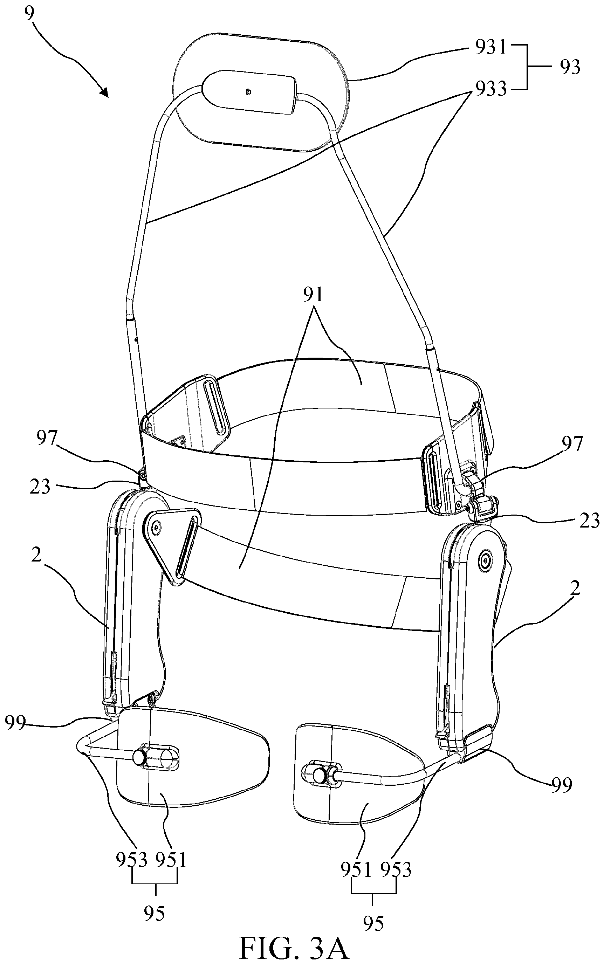

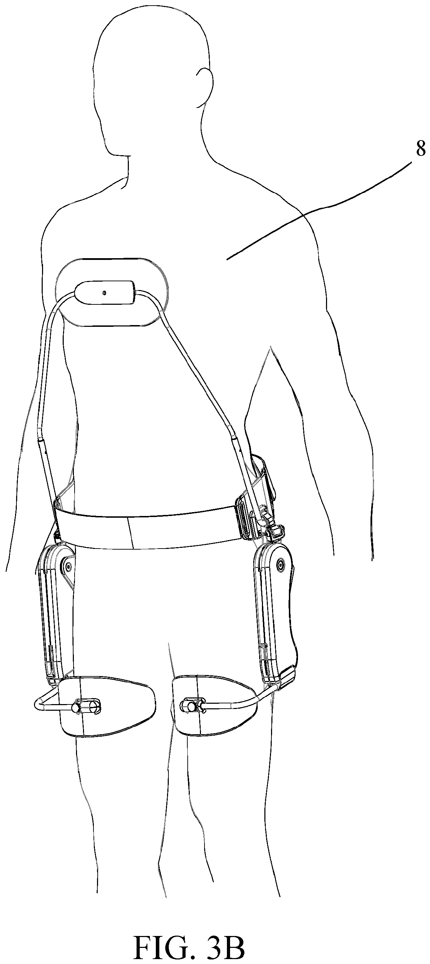

In some embodiments, the supporting device 2 is for use with the assistive equipment to assist the user in performing related motion. Refer to FIG. 3A and FIG. 3B. FIG. 3A is a perspective view of an assistive equipment 9 according to some embodiments of the present disclosure. FIG. 3B is a schematic view of a user 8 using the assistive equipment 9 according to some embodiments of the present disclosure. The assistive equipment 9 comprises two supporting devices 2, a wearing unit 91, a supporting unit 93, and two positioning units 95 corresponding in position to the supporting devices 2.

In some embodiments, the supporting unit 93 is connected to the first positioning element 23 of the supporting device 2 and adapted to support the top half of the body of the user 8. The supporting unit 93 comprises a baffle 931 and a rod 933. The two ends of the rod 933 are connected to the first positioning elements 23 of the supporting devices 2, respectively. The baffle 931 is disposed at the middle segment of the rod 933 and adapted to abut against and support the top half of the body of the user 8.

In some embodiments, two sides of the wearing unit 91 are connected to the supporting devices 2, respectively. The user 8 wears the supporting devices 2, using the wearing unit 91. In some embodiments, the assistive equipment 9 comprises two connecting units 97 disposed on two sides of the wearing unit 9, respectively, and adapted to the supporting unit 91 and the first positioning elements 23 of the supporting devices 2.

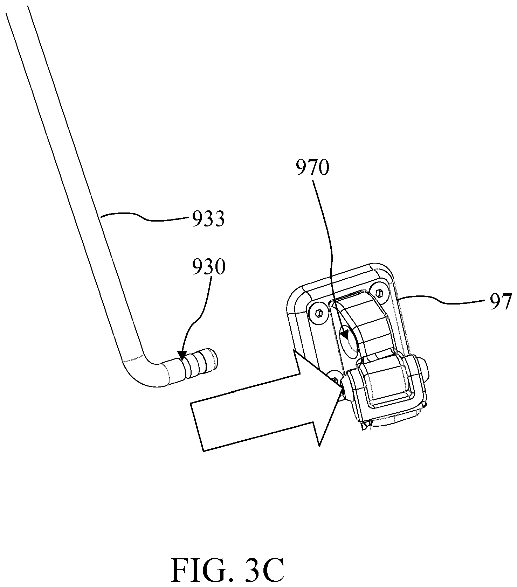

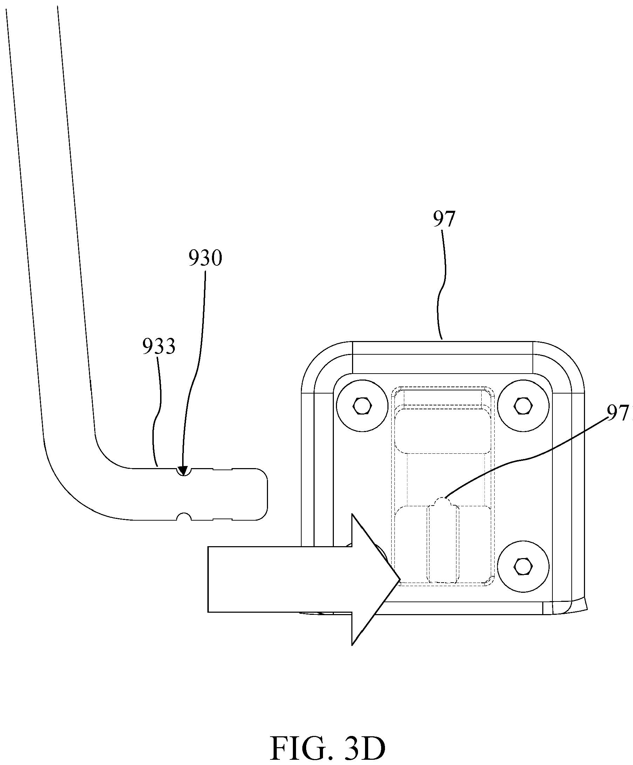

Refer to FIG. 3C and FIG. 3D. FIG. 3C is a schematic perspective view of the supporting unit 93 connected to the connecting unit 97 according to some embodiments of the present disclosure. FIG. 3D is a schematic side view of the supporting unit 93 connected to the connecting unit 97 according to some embodiments of the present disclosure, wherein the internal structure of the connecting unit 97 is demarcated with dashed lines. The connecting unit 97 comprises a receiving hole slot 970 and an engaging element 971. The receiving hole slot 970 receives the end portion of the rod 933 of the supporting unit 93. The engaging element 971 is disposed in the receiving hole slot 970 and adapted to engage with a groove 930 at the end portion of the rod 933 when the receiving hole slot 970 receives the end portion of the rod 933 of the supporting unit 93, so as for the supporting unit 93 to be fixed in place inside the receiving hole slot 970.

In some embodiments, the engaging element 971 has therein a spring so that the engaging element 971 is compressed as soon as the receiving hole slot 970 receives the end portion of the rod 933 of the supporting unit 93. Then, when the groove 930 at the end portion of the rod 933 is aligned with the engaging element 971, the engaging element 971 becomes restored from the compression state to engage with the groove 930 at the end portion of the rod 933.

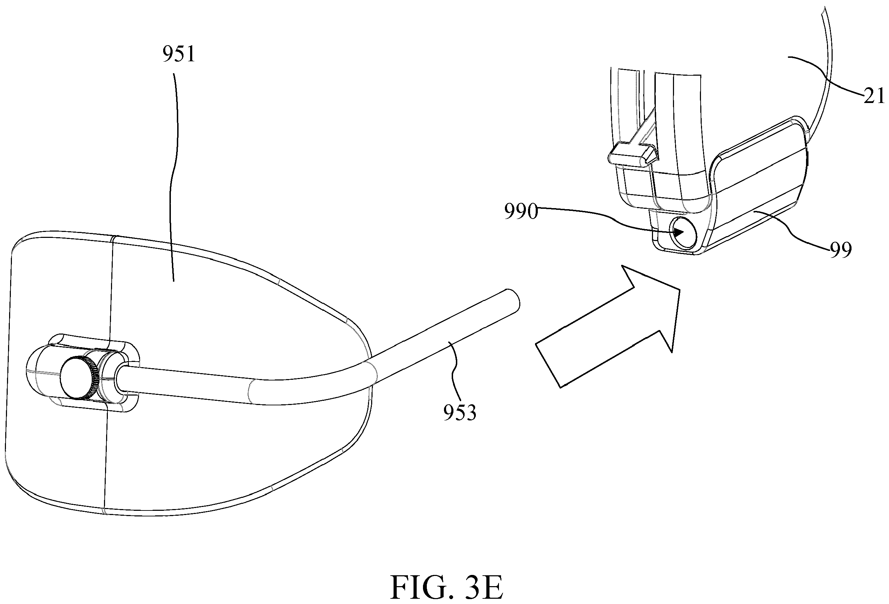

In some embodiments, the assistive equipment 9 comprises two connecting units 99, one of which connects the housing 21 of a supporting device 2 and a positioning unit 95. Referring to FIG. 3E, there is shown a schematic perspective view of the positioning unit 95 connected to the connecting unit 99 according to some embodiments of the present disclosure. The connecting unit 99 is disposed at the housing 21 of the supporting device 2. Each positioning unit 95 comprises a baffle 951 and a rod 953. The baffle 951 is disposed at an end portion of the rod 953 and adapted to abut against the bottom half of the body of the user 8. Each connecting unit 99 comprises a receiving hole slot 990 for receiving and fixing in place the other end portion of the rod 953 of the positioning unit 95.

In some embodiments, both the baffle 931 of the supporting unit 93 and the baffle 951 of the positioning unit 95 are disposed on the front of the body of the user 8. The baffle 931 of the supporting unit 93 abuts against the top half of the body of the user 8. The baffle 951 of the positioning unit 95 abuts against the bottom half of the body of the user 8. Thus, when the user 8 stoops and pushes the baffle 931 of the supporting unit 93 forward, the supporting device 2 located at the waist of the user 8 functions as a pivot, pushing the baffle 951 of the positioning unit 95 toward the bottom half of the body of the user 8. Then, when the user 8 keeps stooping, the supporting unit 93 and the positioning unit 95 rotate relative to each other and about the supporting device 2, because the baffle 951 of the positioning unit 95 is abutting against the bottom half of the body of the user 8.

Then, the user 8 keeps stooping, and the baffle 931 abutting against the top half of the body of the user 8 is pushed forward under gravity; thus, the baffle 931 drives the rod 935 to move, and the rod 935 drives the first positioning element 23 to undergo the rotation R21. At this point in time, the resistance force generated by the elastic element 27 to oppose the rotation R21 of the first positioning element 23 is for use in supporting the top half of the body of the user 8 while the user 8 is stooping.

Then, when the user 8 wants to switch from stooping to standing, the user 8 stops exerting a force on the first positioning element 23. At this point in time, the elastic element 27 presses against the cam 23 to allow the first positioning element 23 to undergo the rotation R22, and the rotation R22 assists the user 8 in switching from stooping to standing.

In conclusion, according to the present disclosure, an assistive equipment with a supporting device can be worn on a user's body and has advantages. First, the user operates the supporting device according to the user's motion, such as stooping, and the supporting device is conducive to the generation and transmission of a supporting force corresponding to the user's motion. Second, when the user restores his or her initial posture from the motion (for example, switches from stooping to standing), the supporting device assists the user in restoring his or her initial posture under the supporting force. Third, as a result, the intuitive use of the assistive equipment is conducive to enhancement of efficiency and ease of use.

The present disclosure is disclosed above by embodiments. However, the embodiments are illustrative of the present disclosure only, but shall not be interpreted as restrictive of the scope of the present disclosure. Hence, all changes and equivalent modifications made by persons skilled in the art to the embodiments shall fall within the scope of the present disclosure. Accordingly, the legal protection for the present disclosure shall be defined by the appended claims.

## Claims

1. A supporting device, comprising:
a housing;
a first positioning element disposed at an end of the housing;
a second positioning element disposed at another end of the housing; and
an elastic element disposed between the first positioning element and the second positioning element,
wherein, when an end of the elastic element is positioned in place by the second positioning element, rotation of the first positioning element about a first pivoting point causes a deformation of the elastic element.

2. The supporting device of claim 1, wherein the second positioning element further comprises an operating portion for operating the second positioning element to allow the second positioning element to be in a first state or a second state.

3. The supporting device of claim 2, wherein an end of the elastic element is positioned in place by the second positioning element when the second positioning element is in the second state.

4. The supporting device of claim 2, wherein the elastic element is slidable between the first positioning element and the second positioning element when the second positioning element is in the first state.

5. The supporting device of claim 2, wherein the second positioning element is disposed at a second pivoting point of the housing, and the operating portion is adapted to operate the second positioning element to allow the second positioning element to rotate about the second pivoting point so as to be in the first state or the second state.

6. The supporting device of claim 1, wherein the elastic element further comprises a first end portion, a second end portion, and an elastomer connected to the first end portion and the second end portion.

7. The supporting device of claim 6, wherein the elastomer comprises a coil spring, a stacked disc spring or a nitrogen spring.

8. The supporting device of claim 1, wherein the first positioning element comprises a cam, when an end of the elastic element is positioned in place by the second positioning element, rotation of the first positioning element about the first pivoting point allows the first positioning element to abut against the elastic element and causes the deformation of the elastic element.

9. An assistive equipment, comprising:
at least one said supporting device of claim 1;
a wearing unit connected to the at least one supporting device to allow the at least one supporting device to be worn on a user's body;
a supporting unit connected to the first positioning element of the at least one supporting device and adapted to support a top half of the body of the user; and
at least one positioning unit connected to the housing of the at least one supporting device and adapted to position the at least one supporting device in place.

10. The assistive equipment of claim 9, further comprising at least one connecting unit for connecting the supporting unit and the first positioning element of the at least one supporting device.

11. The assistive equipment of claim 10, wherein the at least one connecting unit further comprises an engaging element for fixing the supporting unit in place.

12. The assistive equipment of claim 10, wherein the at least one connecting unit is disposed at the wearing unit.

13. The assistive equipment of claim 9, further comprising at least one connecting unit for connecting the at least one positioning unit and the housing of the at least one supporting device.

14. The assistive equipment of claim 13, wherein the at least one connecting unit is disposed at the housing of the at least one supporting device.

15. The assistive equipment of claim 9, wherein the supporting unit further comprises:
a rod connected to the first positioning element of the at least one supporting device; and
a baffle disposed at the rod and adapted to support a top half of the body of the user.
